# EUROPEAN PATENT SPECIFICATION

(11) **EP 2 996 681 B1**
(45) Date of publication and mention of the grant of the patent: **18.12.2019**
(21) Application number: 13785853.6
(22) Date of filing: 31.10.2013
(51) Int. Cl.: A61K 9/48, A61K 31/137

(54) **PHARMACEUTICAL COMPOSITION COMPRISING FINGOLIMOD**
PHARMAZEUTISCHE ZUSAMMENSETZUNG MIT FINGOLIMOD
COMPOSITION PHARMACEUTIQUE CONTENANT DU FINGOLIMOD

(30) Priority: 13.05.2013 WO PCT/EP2013/059807
(43) Date of publication of application: 23.03.2016
(73) Proprietor: Synthon B.V., 6545 CM Nijmegen (NL)
(72) Inventor: ÁLVAREZ FERNÁNDEZ, Lisardo, E-08830 Sant Boi de Llobregat (ES); DALEN van, Frans, NL-6545 CM Nijmegen (NL)
(74) Representative: Mendivil Gil, Maria Dolores
(86) International application number: PCT/EP2013/072767
(87) International publication number: WO 2013/190151

(56) References cited:
- WO-A1-2004/089341
- WO-A2-2009/048993
- WO-A2-2011/131368

## Description

The invention relates to pharmaceutical compositions comprising the compound fingolimod as the active pharmaceutical ingredient.

### BACKGROUND OF THE INVENTION

Fingolimod (often coded as FTY 720), chemically 2-amino-2-[2-(4-octylphenyl)ethyl]-propane-1,3-diol of formula (1) 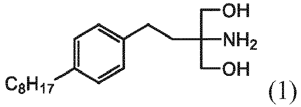 is a pharmaceutically active immunomodulating compound. It is a structural analogue of sphingosine and its pharmaceutical activity is associated with its modulating activity towards sphingosine-1-phosphate (S1P) receptors.

In 2010, fingolimod became the first oral disease-modifying drug approved for treating multiple sclerosis.

Fingolimod itself is a prodrug and gets phosphorylated to the active metabolite (S)-fingolimod phosphate ester by sphingosine kinases in liver cells.

Fingolimod may form stable acid addition salts, of which fingolimod hydrochloride is the most common one.

The approved product sold, e.g., under the trade name Gilenya®, is a hard-shell capsule filled by a powder comprising 0.56 mg of fingolimod hydrochloride (corresponding to 0.5 mg of fingolimod free base) per capsule. The powder further comprises mannitol as a filler and a small amount of magnesium stearate as a lubricant.

The compound was discovered by Yoshitomi and was first disclosed in EP 0627406. In this patent also two basic routes for making it have been described.

Various powderized pharmaceutical compositions comprising fingolimod, which may serve for purposes of oral administration of fingolimod to a patient in need thereof, have been disclosed in prior art documents.

Thus, WO 2004/089341 discloses a solid pharmaceutical composition suitable for oral administration comprising various SIP receptor agonists and a sugar alcohol. The sugar alcohol may act as a diluent, carrier, filler or bulking agent and may suitably be mannitol, maltitol, inositol, xylitol and/or lactitol. It is taught that these compositions do not suffer from the disadvantages of liquid formulations for injection or oral use and have good physicochemical and storage properties. In particular, the compositions show a high level of content uniformity as well as a high stability. The composition may be in the form of a powder, granule, pellet or tablet. In examples of the preferred embodiment, fingolimod hydrochloride is mixed with mannitol and a lubricant and, optionally with a binder such as HPC or HPMC, milled and/or granulated.

Apparently, the composition of the marketed product Gilenya® falls within the scope of the compositions that are disclosed and claimed in WO 2004/089341.

WO 2008/037421 provides formulations comprising a SIP receptor modulator and adapted for oral administration in solid form, which can be easily swallowed, e.g. by children or elderly patients. The invention provides dosage forms, which disintegrate rapidly in the mouth and do not depend on the presence of a taste masking agent or on the presence of water for washing down the dosage form. It also provides compositions comprising an SIP receptor modulator, wherein the composition is coated by a coating comprising one or more polymer resins and one or more metal oxides. In further, it provides a composition comprising an SIP receptor modulator and microcrystalline cellulose in the absence of a sugar alcohol.

WO 2009/048993 teaches that various SIP receptor modulators comprising an aminopropane-1,3-diol group (such as, e.g., fingolimod) are not easy to formulate in a solid oral formulation; only a limited number of excipients are potentially feasible with such amino diols. In particular, reducing sugars are not considered suitable due to the danger of a Maillard reaction with the amino-group. Thus, according to WO 2009/048993, the only suitable fillers providing stable blends with the aminopropane-1-3-diol based SIP receptor modulator are lactose, lactose monohydrate, maize starch, mannitol, xylitol, sorbitol, sucrose, microcrystalline cellulose, dibasic calcium phosphate, maltodextrin and gelatin. This selection was supported by stability testing of various blends comprising fingolimod and the excipient for 1 month at 50°C. In addition, several binders, disintegrants, lubricants, flow regulators, matrix formers, plasticizers, flavouring agents, and sweeteners were marked as suitable for making solid oral formulations.

In summary, the prior art teachings indicate that S1P receptor modulators comprising an aminopropane-1,3-diol group are not easily formulated into a stable solid oral formulation. Apparently, this is because of the reactivity of the aminopropane-1,3-diol group. Only a limited number of suitable pharmaceutical excipients, particularly fillers, have been accordingly found. Thus, it will be beneficial to provide an alternative and/or improved composition for oral administration of fingolimod, which is stable and has good handling properties in making pharmaceutical dosage forms for oral administration.

### SUMMARY OF THE INVENTION

The present invention relates to a pharmaceutical composition suitable for oral administration of fingolimod, a salt thereof and/or an ester thereof, which composition exhibits improved stability upon long-term storage and has advantageous handling properties in making orally administrable final dosage forms such as capsules or tablets.

In one aspect, the present invention relates to a pharmaceutical composition comprising fingolimod or a pharmaceutically acceptable salt or ester thereof, tripotassium citrate and, optionally, a lubricant. The composition is preferably formulated in the form of a powder, granulate, or compressed tablet for oral administration. The composition may further comprise a glidant, preferably colloidal silicon dioxide.

Preferably, the pharmaceutically acceptable salt of fingolimod is fingolimod hydrochloride.

Preferably, the pharmaceutically acceptable ester of fingolimod is (S)-fingolimod phosphate.

Preferably, the lubricant is magnesium stearate.

The weight ratio of tripotassium citrate to fingolimod or a pharmaceutically acceptable salt or ester thereof is from 99.5:0.5 to 80:20, preferably from 99:1 to 90:10, calculated as fingolimod free base.

Preferably, the composition does not comprise a binder.

Preferably, the composition consists of fingolimod or a pharmaceutically acceptable salt or ester thereof, tripotassium citrate, silicon dioxide and, optionally, a lubricant.

In a second aspect, the invention relates to a process for making said pharmaceutical composition, comprising the steps of
a) mixing fingolimod or a pharmaceutically acceptable salt or ester thereof with tripotassium citrate;
b) optionally, screening, milling and/or granulating the mixture obtained in the step a); and
c) optionally mixing the mixture from step a) or from step b) with a lubricant.

In a third aspect, the invention relates to the use of tripotassium citrate for making pharmaceutical compositions comprising fingolimod or a pharmaceutically acceptable salt or ester thereof.

In a fourth aspect, the invention relates to a composition comprising fingolimod or a pharmaceutically acceptable salt or ester thereof, tripotassium citrate and, optionally, a lubricant, for use as a medicament, preferably in treating or preventing a disease or condition treatable by fingolimod, more preferably for treating multiple sclerosis.

### DETAILED DESCRIPTION OF THE INVENTION

The present invention relates to an oral pharmaceutical composition, and oral dosage forms, comprising fingolimod. "Fingolimod" is a generically used name for 2-amino-2-[2-(4-octylphenyl)ethyl]-propane-1,3-diol and will be so used throughout this specification, unless expressly stated differently.

Fingolimod comprises a basic amino-group and may accordingly form acid addition salts with organic or inorganic acids. In accordance with its intended use, pharmaceutically acceptable acids are preferred. Examples of pharmaceutically acceptable acids are, without limitation, hydrochloric, hydrobromic, sulfuric, phosphoric, nitric, formic, acetic, propionic, oxalic, malonic, maleic, fumaric, lactic, citric, malic, tartaric, methane sulfonic, benzene sulfonic, naphthalene disulfonic acid, etc. A preferred pharmaceutically acceptable salt is fingolimod hydrochloride.

Fingolimod also comprises two hydroxyl groups and may accordingly form (an) ester(s) with inorganic or organic acid(s). In accordance with its intended use, pharmaceutically acceptable acids are preferred. The acids listed in the preceeding paragraph may be advantageously employed, without any restriction to other suitable acids. If only one hydroxyl group is substituted, a compound with a chiral carbon is formed. Accordingly, the esters may be of (R)-configuration, (S)-conformation or may be a racemic mixture thereof. Any of such possibilities is covered by the term "ester" in accordance with the present invention, unless specifically stated otherwise. A preferred pharmaceutically acceptable ester is fingolimod-phosphate ester, preferably (S)-fingolimod-phosphate ester.

Solid state fingolimod, a salt and/or ester thereof may exist as a crystalline or an amorphous material. Crystalline materials may exist in different polymorphic modifications. In addition, they may be substantially anhydrous or may exist in the form of a hydrate and/or a solvate. Any such modifications are included within the terms "fingolimod", "fingolimod salt" and "fingolimod ester" throughout this specification.

Fingolimod, a salt thereof or an ester thereof are either commercially available or may be obtained by processes known in the art.

The essential features of compositions of the present invention are fingolimod or a salt or an ester thereof, and tripotassium citrate. Tripotassium citrate is a known compound approved in particular for use in food and as a pharmaceutical agent in the treatment of gout, arrhythmia and cystinuria. Its use as a pharmaceutical excipient in making oral dosage forms appears not to be common. Tripotassium citrate is a stable water soluble compound and may be obtained either commercially or by processes known in the art. Tripotassium citrate exists in an anhydrated (anhydrous) form and in a hydrated (e.g. monohydrate) form. Both forms are encompassed by the term "tripotassium citrate".

It was found out by the present inventor that tripotassium citrate formed stable compositions with fingolimod or salts or esters thereof. The stability of such compositions in long-term storage tests is at least comparable with that of similar compositions with mannitol, which is considered in the prior art as the most suitable filler for formulating fingolimod into oral pharmaceutical compositions, and it is superior to many other earlier suggested fillers. The compositions of the present invention exhibit good handling properties, e.g. flowability, content uniformity etc. for making powders or granulates for both direct oral administration or for tabletting. Furthermore, tripotassium citrate efficiently masks the unpleasant taste of fingolimod free base without providing a sweet taste as, e.g., mannitol and similar sugar alcohols do.

Accordingly, the present invention provides for a solid composition for oral administration comprising fingolimod or a salt or ester thereof and tripotassium citrate and, optionally, a lubricant wherein the weight ratio of tripotassium citrate to fingolimod or a salt or ester thereof is from 99.5:0.5 to 80:20, more preferably from 99:1 to 90:10, calculated as fingolimod free base.

In yet another advantageous embodiment, the composition of the present invention comprises tripotassium citrate as the single excipient serving as a filler. Thus, in particular, the composition does not comprise a sugar alcohol such as mannitol and/or sorbitol, a phosphate such as calcium phosphate and/or a cellulose such as microcrystalline cellulose.

The composition preferably further comprises a lubricant/glidant, which improves the flow of the composition and minimizes adherence to walls of equipment. Suitable lubricants/glidants include stearic acid, magnesium stearate, calcium stearate, zinc stearate, glyceryl palmitostearate, sodium stearyl fumarate, hydrogenated vegetable oil, sodium lauryl sulfate, magnesium oxide, colloidal silicon dioxide, talc, poloxamer or a mixture of any of the above. Preferably, the lubricant is a stearate, most preferably magnesium stearate. Preferably, the glidant is colloidal silicon dioxide. The amount of lubricant/glidant is typically about 25-100 weight % with respect to the weight of the fingolimod in the mixture, calculated as fingolimod free base.

In an advantageous embodiment, the composition does not comprise any excipient other than tripotassium citrate, colloidal silicon dioxide and, optionally, a lubricant. Thus, in a specific aspect, the invention provides for a pharmaceutical composition consisting of fingolimod or a pharmaceutically acceptable salt or ester thereof, tripotassium citrate, colloidal silicon dioxide and, optionally, a lubricant.

The use of other excipients is not excluded, however. In particular, the composition may optionally comprise a binder. Suitable binders may include a cellulose or a cellulose derivative, e.g. hydroxypropyl cellulose or hydroxypropylmethyl cellulose. The amount of binder may typically be from 0.05 to 5 weight % with respect to the weight of the fingolimod/tripotassium citrate mixture.

The composition of the present invention may be formulated to final dosage forms for oral administration. Such dosage form may comprise a dose of powder or granulate comprising the composition of the invention, which is filled in a hard-shell capsule or in a sachet. Such dosage form may also comprise the composition of the invention compressed into a tablet. The tablet is preferably a swallowable tablet. It may optionally be coated with a film coat comprising, in essence, any suitable inert coating material known in the art. The dosage form advantageously comprises a unit dose of fingolimod, which may be from 0.1 to 2 mg of fingolimod, preferably 0.1, 0.2, 0.25, 0.5, 1.0 or 2.0 mg of fingolimod, calculated as the free base. In total, a single dosage form may advantageously comprise from 10 to 200 mg of the composition. A preferred dosage form is a hard-shell capsule filled with a dose of the composition of the present invention in the form of a powder or a granulate. The hard-shell capsule may advantageously be made from gelatin.

In another aspect, the invention relates to a process for producing a pharmaceutical composition as defined hereinabove, comprising the steps of
a) mixing fingolimod or a pharmaceutically acceptable salt or ester thereof with tripotassium citrate;
b) optionally, screening, milling and/or granulating the mixture obtained in the step a); and
c) optionally mixing the mixture from step a) or step b) with a lubricant.

More particularly, the composition as discussed above may be produced by this process.

### Sub a)

Fingolimod or a pharmaceutically acceptable salt or ester thereof, typically fingolimod hydrochloride or fingolimod phosphate ester may optionally be milled and/or pre-screened before mixing in step a) in order to remove lumps. Advantageously, the particles of the treated product pass a screen with 400-800 µm (0.4-0.8 mm) mesh size. Accordingly, tripotassium citrate may be treated in the same manner.

The weight ratio of tripotassium citrate to fingolimod or a salt or ester thereof is advantageously from 99.5:0.5 to 80:20, more preferably from 99:1 to 90:10, calculated as fingolimod free base.

The mixing step a) may advantageously comprise dry or wet mixing of components in any suitable blender at, e.g., 100 to 400 revolutions per minute. The mixing step a) also comprises mixing *per partes,* i.e. when the fingolimod component is mixed first with a small amount of tripotassium citrate, e.g. from 5 to 50 per cent of the total charge of tripotassium citrate, in order to form a pre-mix. Subsequently the remaining amount of tripotassium citrate is added to the pre-mix in one or more doses.

While being not the preferred variant, step a) also may comprise the step of adding a binder solution, whenever the binder is appropriate. A list of suitable binders has been provided above. Typically, the binder is added in a solution in an appropriate liquid, e.g. in water, alcohol or a mixture of both, and the mixture is subjected to a granulation in step b). Alternatively, the binder is added to the mix dry and the granulation liquid is added in the granulation step b).

In the preferred variant, indeed, no binder is used.

### Sub b)

The purpose of the optional step b) is to suitably modify the physical properties of the mixture from step a) for its formulation in medicinal dosage forms. If appropriate, the mixture may be screened, advantageously through a screen of mesh size of about 400-800 µm, and/or optionally milled on a suitable mill. If appropriate, the mixture may be granulated in a suitable high sheer mixer-granulator and/or in a fluid bed granulator. A drying step may be included as well, e.g., within the granulation process.

### Sub c)

Mixing with a suitable lubricant, preferably with a stearate and most preferably with magnesium stearate, provides a free-flowing particulate composition suitable for use in making the solid-state final forms as discussed above. The lubricant is preferably pre-screened, e.g. with a screen of 800-900 µm mesh size.

In accordance with the invention, the free-flowing composition obtained in step c), which is typically in the form of a powder or granulate is then formulated into medicinal final dosage forms. Suitable final dosage forms have been discussed above.

In summary, there has been manifested a suitability and advantage of using tripotassium citrate in making fingolimod-comprising pharmaceutical compositions. Accordingly, a specific aspect of the present invention relates to a novel and advantageous use of this compound, namely the use of tripotassium citrate for making pharmaceutical compositions comprising fingolimod or a pharmaceutically acceptable salt or ester thereof.

The pharmaceutical compositions of the present invention and/or final dosage forms comprising them are useful, for treating or preventing a disease or condition treatable by fingolimod. The "disease or condition treatable by fingolimod" as used herein may comprise, without limitation:
- treatment and/or prevention of organ or tissue transplant rejection, particularly the treatment of acute or chronic allo- and xenograft rejection or the transplantation of insulin producing cells;
- treatment and/or prevention of autoimmune disease or of inflammatory conditions, e.g. multiple sclerosis, arthritis (e.g. rheumatoid arthritis), inflammatory bowel disease, hepatitis etc.;
- treatment and/or prevention of viral myocarditis and viral diseases caused by viral myocarditis, including hepatitis and AIDS.

The invention will be further illustrated by the following non-limiting examples.

### Examples

### Example 1. Pharmaceutical capsule dosage form comprising 0.5 mg of fingolimod

**Composition**

| **Ingredient** | **Per capsule** | **Percentage w/w** |
|---|---|---|
| Fingolimod hydrochloride | 0.559 mg* | 1.165% |
| Tripotassium citrate monohydrate POWDER | 46.823 mg | 97.548% |
| Colloidal silicon dioxide (Aerosil) | 0.378 mg | 0.787% |
| Magnesium stearate | 0.240 mg | 0.500% |
| Total | 48.000 mg | 100.00% |

| | | |
|---|---|---|
| * Equivalent to 0.5 mg of fingolimod free base | | |

### Process

Tripotassium citrate monohydrate POWDER and Aerosil were blended and sieved through a 0.8 mm mesh size. Fingolimod hydrochloride and part of the tripotassium citrate monohydrate/Aerosil blend were blended and sieved through a 1.1 mm mesh size. Then progressive drug substance dilutions with the rest of the tripotassium citrate monohydrate/Aerosil blend were performed. After each one of the additions the blend was mixed. Prior to the addition the tripotassium citrate monohydrate/Aerosil blend was sieved through the 1.1 mm mesh size to avoid any fingolimod loss. Magnesium stearate was sieved using a 0.8 mm mesh size, then added to the powder blend and mixed. The final blend was filled into hard gelatin capsules number 3.

### Compositions

A capsule comprising the composition of fingolimod hydrochloride with tripotassium citrate monohydrate POWDER, colloidal silicon dioxide and magnesium stearate has been prepared according to Example 1 (Composition A). Similar capsules comprising, instead of tripotassium citrate monohydrate POWDER, the same amount of a different grade of tripotassium citrate were prepared accordingly:
Composition B 98.335% Tripotassium citrate monohydrate POWDER (no colloidal silicon dioxide in the formulation)
Composition C 98.335% Tripotassium citrate anhydrous (no colloidal silicon dioxide in the formulation)
Composition D 97.548% Tripotassium citrate monohydrate FINE CRYSTAL (and 0.8% colloidal silicon dioxide)

The capsules were packed in Alu-Alu blister and subjected to stability testing at 25°C/60%RH, 30°C/65%RH and 40°C/75%RH in a thermostated chamber.

The contents of fingolimod as well as of impurities were determined by HPLC. The results of the stability study were as follows:

| | | 25 °C / 60% RH; time (months) | | | |
|---|---|---|---|---|---|
| | | 0(%) | 2 (%) | 3 (%) | 6 (%) |
| **Composition A** | Assay | 100.50 | - | 98.3 | 98.3 |
| | Impurity (total) | ≤ 0.1 | - | ≤ 0.1 | ≤ 0.1 |
| **Composition B** | Assay | 105.1 | - | 100.5 | |
| | Impurity (total) | ≤ 0.1 | - | ≤ 0.1 | |
| **Composition C** | Assay | 102.0 | 97.2 | 98.0 | 95.5 |
| | Impurity (total) | ≤ 0.1 | ≤ 0.1 | ≤ 0.1 | ≤ 0.1 |
| **Composition D** | Assay | 82.5 | - | 79.3 | |
| | Impurity (total) | ≤ 0.1 | - | ≤ 0.1 | |

| | | **30 °C / 65% RH; time (months)** | | | | |
|---|---|---|---|---|---|---|
| | | **0 (%)** | **1 (%)** | **2 (%)** | **3 (%)** | **4 (%)** |
| **Composition A** | Assay | 100.50 | 98.7 | 98.5 | 99.0 | 98.4 |
| | Impurity (total) | ≤ 0.1 | ≤ 0.1 | 0.30 | ≤ 0.1 | ≤ 0.1 |
| **Composition B** | Assay | 105.1 | 106.9 | 102.0 | 104.8 | |
| | Impurity (total) | ≤ 0.1 | ≤ 0.1 | ≤ 0.1 | ≤ 0.1 | |
| **Composition C** | Assay | 102.0 | | | | |
| | Impurity (total) | ≤ 0.1 | | | | |
| **Composition D** | Assay | 82.5 | 79.0 | 79.6 | 80.9 | |
| | Impurity (total) | ≤ 0.1 | ≤ 0.1 | ≤ 0.1 | ≤ 0.1 | |

| | | **40 °C / 75% RH; time (months)** | | | | |
|---|---|---|---|---|---|---|
| | | **0 (%)** | **1 (%)** | **2 (%)** | **3 (%)** | **6 (%)** |
| **Composition A** | Assay | 100.50 | 99.7 | 99.2 | 99.6 | 100.6 |
| | Impurity (total) | ≤ 0.1 | ≤ 0.1 | ≤ 0.1 | 0.3% | ≤ 0.1 |
| **Composition B** | Assay | 105.1 | - | 101.7 | 106.1 | |
| | Impurity (total) | ≤ 0.1 | ≤ 0.1 | ≤ 0.1 | ≤ 0.1 | |
| **Composition C** | Assay | 102.0 | 96.9 | 94.1 | 96.9 | 95.8 |
| | Impurity (total) | ≤ 0.1 | ≤ 0.1 | ≤ 0.1 | ≤ 0.1 | ≤ 0.1 |
| **Composition D** | Assay | 82.5 | 80.9 | 80.2 | 82.7 | |
| | Impurity (total) | ≤ 0.1 | ≤ 0.1 | ≤ 0.1 | ≤ 0.1 | |

## Claims

1. A pharmaceutical composition comprising fingolimod or a pharmaceutically acceptable salt or ester thereof, tripotassium citrate and, optionally, a lubricant wherein the weight ratio of tripotassium citrate to fingolimod or a salt or ester thereof is from 99.5:0.5 to 80:20, calculated as fingolimod free base.

2. The composition according to claim 1, wherein the pharmaceutically acceptable salt of fingolimod is fingolimod hydrochloride.

3. The composition according to claim 1, wherein the pharmaceutically acceptable ester of fingolimod is (S)-fingolimod phosphate.

4. The composition according to any one of claims 1 to 3, wherein the lubricant is a stearate, preferably magnesium stearate.

5. The composition according to any one of claims 1 to 4, wherein the composition does not comprise a binder.

6. The composition according to any one of claims 1 to 5 further comprising a glidant, preferably colloidal silicon dioxide.

7. The composition according to any one of claims 1 to 6, wherein the composition consists of fingolimod or a pharmaceutically acceptable salt or ester thereof, tripotassium citrate, colloidal silicon dioxide and, optionally, a lubricant.

8. The composition according to any one of claims 1 to 7, wherein the composition is formulated in the form of a powder, granulate or compressed tablet for oral administration.

9. A pharmaceutical dosage form comprising the composition according to any one of claims 1 to 8.

10. The dosage form according to claim 9 comprising from 0.1 to 2 mg of fingolimod, preferably 0.1, 0.2, 0.25, 0.5, 1.0 or 2.0 mg of fingolimod, calculated as the free base.

11. The dosage form according to claim 9 or 10 in the form of a hard-shell capsule.

12. A process for producing a pharmaceutical composition according to any one of claims 1 to 8, comprising the steps of
a) mixing fingolimod or a pharmaceutically acceptable salt or ester thereof with tripotassium citrate;
b) optionally, screening, milling and/or granulating the mixture obtained in step a); and
c) optionally mixing the mixture from step a) or from step b) with a lubricant.

13. The process according to claim 12 further comprising adding colloidal silicon dioxide to the mixture of step a).

14. The process according to claim 12 or 13 further comprising a step of formulating the composition into a pharmaceutical dosage form, preferably a dosage form according to claim 10 or 11.

## Patentansprüche

1. Pharmazeutische Zusammensetzung, die Fingolimod oder ein pharmazeutisch akzeptables Salz oder Ester davon, Trikaliumcitrat und, optional, ein Schmiermittel umfasst, wobei das Gewichtsverhältnis von Trikaliumcitrat zu Fingolimod oder einem Salz oder Ester davon von 99.5:0.5 bis 80:20, berechnet als freie Fingolimod-Basis, ist.

2. Zusammensetzung nach Anspruch 1, wobei das pharmazeutisch akzeptable Salz von Fingolimod Fingolimod-Hydrochlorid ist.

3. Zusammensetzung nach Anspruch 1, wobei das pharmazeutisch akzeptable Ester von Fingolimod (S)-Fingolimod-Phosphat ist.

4. Zusammensetzung nach einem der Ansprüche 1 bis 3, wobei das Schmiermittel ein Stearat, bevorzugt Magnesiumstearat, ist.

5. Zusammensetzung nach einem der Ansprüche 1 bis 4, wobei die Zusammensetzung kein Bindemittel umfasst.

6. Zusammensetzung nach einem der Ansprüche 1 bis 5, ferner umfassend ein Gleitmittel, bevorzugt kolloidales Siliziumdioxid.

7. Zusammensetzung nach einem der Ansprüche 1 bis 6, wobei die Zusammensetzung aus Fingolimod oder einem pharmazeutisch akzeptablen Salz oder Ester davon, Trikaliumcitrat, kolloidalem Siliziumdioxid und, optional, einem Schmiermittel besteht.

8. Zusammensetzung nach einem der Ansprüche 1 bis 7, wobei die Zusammensetzung in der Form eines Pulvers, eines Granulats oder einer komprimierten Tablette für die orale Verabreichung formuliert ist.

9. Pharmazeutische Dosierungsform, umfassend die Zusammensetzung nach einem der Ansprüche 1 bis 8.

10. Dosierungsform nach Anspruch 9, umfassend von 0.1 bis 2 mg Fingolimod, bevorzugt 0.1, 0.2, 0.25, 0.5, 1.0 oder 2.0 mg Fingolimod, berechnet als die freie Basis.

11. Dosierungsform nach Anspruch 9 oder 10 in der Form einer Hartkapsel.

12. Prozess zur Herstellung einer pharmazeutischen Zusammensetzung nach einem der Ansprüche 1 bis 8, umfassend die Schritte des
a) Mischens von Fingolimod oder eines pharmazeutisch akzeptablen Salzes oder Esters davon mit Trikaliumcitrat;
b) optional Sieben, Mischen und/oder Granulieren der in Schritt a) erhaltenen Mischung; und
c) optional Mischen der Mischung von Schritt a) oder von Schritt b) mit einem Schmiermittel.

13. Prozess nach Anspruch 12, ferner umfassend das Hinzufügen von kolloidalem Siliziumdioxid zur Mischung von Schritt a).

14. Prozess nach Anspruch 12 oder 13, ferner umfassend einen Schritt des Formulierens der Zusammensetzung in einer pharmazeutischen Dosierungsform, bevorzugt einer Dosierungsform nach Anspruch 10 oder 11.

## Revendications

1. Composition pharmaceutique comprenant du fingolimod ou un sel ou ester de celui-ci pharmaceutiquement acceptable, le citrate de tripotassium et, facultativement, un lubrifiant, dans laquelle le rapport de poids du citrate de tripotassium au fingolimod ou un sel ou ester de celui-ci est de 99.5:0.5 à 80:20, calculé comme base libre de fingolimod.

2. Composition selon la revendication 1, dans laquelle le sel pharmaceutiquement acceptable du fingolimod est le chlorhydrate de fingolimod.

3. Composition selon la revendication 1, dans laquelle l'ester pharmaceutiquement acceptable du fingolimod est le phosphate (S)-fingolimod.

4. Composition selon l'une quelconque des revendications 1 à 3, dans laquelle le lubrifiant est un stéarate, de préférence du stéarate de magnésium.

5. Composition selon l'une quelconque des revendications 1 à 4, dans laquelle la composition ne comprend pas de liant.

6. Composition selon l'une quelconque des revendications 1 à 5, comprenant en outre un agent de glissement, de préférence du dioxyde de silicium colloïdal.

7. Composition selon l'une quelconque des revendications 1 à 6, dans laquelle la composition comprend du fingolimod ou un sel, ou un ester pharmaceutiquement acceptable, du citrate tripotassique, du dioxyde de silicium colloïdal et, facultativement, d'un lubrifiant.

8. Composition selon l'une quelconque des revendications 1 à 7, dans laquelle la composition est formulée en forme de poudre, de granulés ou comprimé à administrer par voie orale.

9. Forme de dosage pharmaceutique comprenant la composition selon l'une quelconque des revendications 1 à 8.

10. Forme de dosage selon la revendication 9, comprenant de 0.1 à 2 mg de fingolimod, de préférence 0.1, 0.2, 0.25, 0.5, 1.0 ou 2.0 mg de fingolimod, calculé comme base libre.

11. Forme de dosage selon la revendication 9 ou 10 ayant la forme d'une gélule dure.

12. Procédé de production d'une composition pharmaceutique selon l'une quelconque des revendications 1 à 8, comprenant les étapes :
a) de mélange du fingolimod ou d'un sel ou d'un ester pharmaceutiquement acceptable de ceux-ci avec le citrate de tripotassium ;
b) éventuellement, le tamisage, le broyage et/ou le granulage du mélange obtenu à l'étape a) ; et
c) éventuellement mélanger le mélange de l'étape a) ou de l'étape b) avec un lubrifiant.

13. Procédé selon la revendication 12, comprenant en outre l'ajout du dioxyde de silicium colloïdal au mélange de l'étape a).

14. Procédé selon la revendication 12 ou 13 comprenant en outre une étape de formulation de la composition en une forme pharmaceutique, de préférence une forme galénique selon la revendication 10 ou 11.
